# EUROPEAN PATENT APPLICATION

(11) **EP 2 165 674 A1**
(43) Date of publication of application: **24.03.2010**
(21) Application number: 08016373.6
(22) Date of filing: 17.09.2008
(51) Int. Cl.: A61C 19/04, A61B 1/24, A61B 1/05

(54) **Optical dental device and method for periodontal pocket examination**

(71) Applicant: Orangedental GmbH & Co. KG, 88400 Biberach (DE)
(72) Inventor: Piernitzki, Bernhard, 19089 Kobande (DE)
(74) Representative: Cramphorn, Conrad

(57) **Abstract**

An optical dental device (1) for periodontal pocket examination, a dental examination system including such a device and a method for operating such a dental examination system are disclosed. The optical dental device (1) comprises an elongate handle (2), a camera integrated in the distal end of the elongate handle (2), and an arm member (7) connected to the elongate handle (2) so that a distal elongate portion (7c) of the arm member (7) extends at least partially across the field of view of the camera (2). A plurality of visual markings axe disposed on and along the distal elongate portion (7c) of the arm member (7) providing information about the distance of a point of the distal elongate portion (7c) of the arm member (7) from the tip (7d) thereof. The dental examination system comprises the optical dental device (1), an image processing unit (10) for receiving and processing images captured by the camera of the optical dental device (1), and a monitor (12) for displaying images captured by the camera of the optical dental device (1).

## Description

### FIELD OF THE INVENTION

The present invention relates to an optical dental device, a dental examination system including such a device and a method of operating the same, and in particular the present invention relates to a hand-held optical dental device for intra-oral examinations and a dental examination system including such a device for determining the periodontal disease index by measuring the depth of periodontal pockets.

### BACKGROUND OF THE INVENTION

Periodontitis is an inflammatory disease affecting the tissue that surrounds and supports the teeth. Periodontitis involves progressive loss of the alveolar bone around the teeth and may lead to periodontal pockets and eventually the loosening and subsequent loss of teeth if left untreated. Periodontitis is caused by a convergence of bacteria that adhere to and grow on the tooth's surfaces, along with an overly aggressive immune system response against these bacteria.

The periodontal disease index is an index used for estimating the degree of periodontitis based on the measurement of six representative teeth for gingival inflammation, pocket depth, calculus and plaque, attrition, mobility and lack of contact. Conventionally, a standardised dental device recommended by the World Health Organisation (a so-called WHO probe) is used to measure the depth of periodontal pockets. The WHO probe comprises a bead having a diameter of 0,5 mm that is located at a substantially L-shaped distal end of a handle of the probe. For measuring the depth of a periodontal pocket the distal end of the WHO probe is inserted into such a pocket, until the bead abuts the gum tissue at the bottom of the pocket. A couple of optical markings located on the distal end of the probe at different distances from the bead allow a dentist to visually determine how far the distal end of the WHO including the bead extends into the periodontal pocket.

The German patent application DE 101 21 676 A1 discloses a system having such a needle type periodontal probe. A miniature thermo-sensor is located in the distal tip of the probe. The system further includes an electronic unit with first and second inputs, whereby the first input is provided for connection of the miniature thermo-sensor and the second is connected to a reference temperature from a patient temperature measurement unit.

The object of the present invention is to provide for an improved dental device for measuring the depth of periodontal pockets allowing a dentist a more comfortable examination of a patient's teeth and providing for better, more objective results.

### SUMMARY OF THE INVENTION

According to a first aspect of the invention an optical dental device is provided comprising an elongate handle. A camera is integrated into or connected to the elongate handle at the distal end thereof. Preferably, the camera comprises a lens, an image sensor and a control unit. An arm member is connected with its proximal end to the elongate handle in such a way that a distal elongate portion of the arm member extends at least partially across the field of view of the camera. Preferably, the arm member is substantially U-shaped having a first proximal leg and a second distal leg so that the second distal leg of the substantially U-shaped arm member extends across the field of view of the camera. A plurality of visual markings are disposed on and along the distal elongate portion of the arm member providing information about the distance of a point of the distal elongate portion of the arm member from the tip thereof.

According to a second aspect of the invention the above-mentioned optical dental device is part of a dental examination system, further comprising a monitor for displaying an image captured by the camera, an image processing unit for analyzing and processing an image captured by the camera and/or a database for storing an image captured by the camera as well as information derived from such an image.

According to a third aspect of the present invention a method is provided for operating the above-mentioned dental examination system including the optical dental device. The method comprises the steps of: displaying on a monitor the insertion of the tip of the distal elongate portion of the arm member in a periodontal pocket of a tooth being examined; triggering the determination of the depth of the periodontal pocket of the tooth being examined; and determining the pocket depth by means of the image processing unit and assigning the determined value to the tooth being examined.

In use, the tip of the distal portion of the arm member can be inserted into a periodontal pocket of a patient until the tip abuts the bottom of the periodontal pocket. This action is continuously captured by the camera and can be displayed on a monitor in communication with the camera and/or the image processing unit so that the dentist and possibly also the patient can view the examination process on the monitor.

The image captured by the camera is preferably transmitted to an image processing unit, such as a suitably configured PC. This transmission can be configured as a wired or a wireless transmission. To this end the optical dental device can include a suitably configured transmission member, such as an antenna, and the image processing unit a correspondingly configured receiving member.

Preferably, the image processing unit is configured to automatically analyze the images provided by the camera. The image processing unit is capable of determining the depth of a periodontal pocket by analyzing an image that shows a portion of the visual markings disposed on and along the distal elongate portion of the arm member being covered by the gum of the periodontal pocket.

Preferably, the dentist can trigger a measurement, i.e. the analysis of an image by the image processing unit, by selecting an image using selection means, such as a button provided on the elongate handle of the optical dental device according to the present invention or an audible command to be received by the image processing unit.

Alternatively, a measurement can be triggered automatically. To this end, the optical dental device according to the present invention, preferably, includes means for measuring the force acting on the tip of the distal portion of the arm member. Once these force measurement means experience a resistance force due to the abutment of the tip of the distal elongate portion of the arm member with the bottom of a periodontal pocket above a certain predefined threshold the depth of the periodontal pocket will be determined by the image processing unit. Such force measurement means can be provided e.g. by a sensor that is based on the piezoelectric effect and that is located in the tip of the distal elongate portion of the arm member. In such an embodiment, the tip of the distal elongate portion of the arm member is made from an elastic material. Preferably, the tip of the distal elongate portion of the arm member is configured as a bead with a diameter of about 0,5 mm.

Further preferred embodiments of the invention are defined in the dependent claims.

### BRIEF DESCRIPTION OF THE DRAWINGS

Figure 1 shows schematically a preferred embodiment of an optical dental device according to the present invention in the context of a dental examination system.
Figure 2 shows in the form of a flow diagram a preferred method of operating the dental examination system including an optical dental device according to the present invention.

### DETAILED DESCRIPTION OF PREFERRED EMBODIMENTS

The present invention will now be further described by defining different aspects of the invention generally outlined above in more detail. Each aspect so defined may be combined with any other aspect or aspects unless clearly indicated to the contrary. In particular, any feature indicated as being preferred or advantageous may be combined with any other feature or features indicated as being preferred or advantageous.

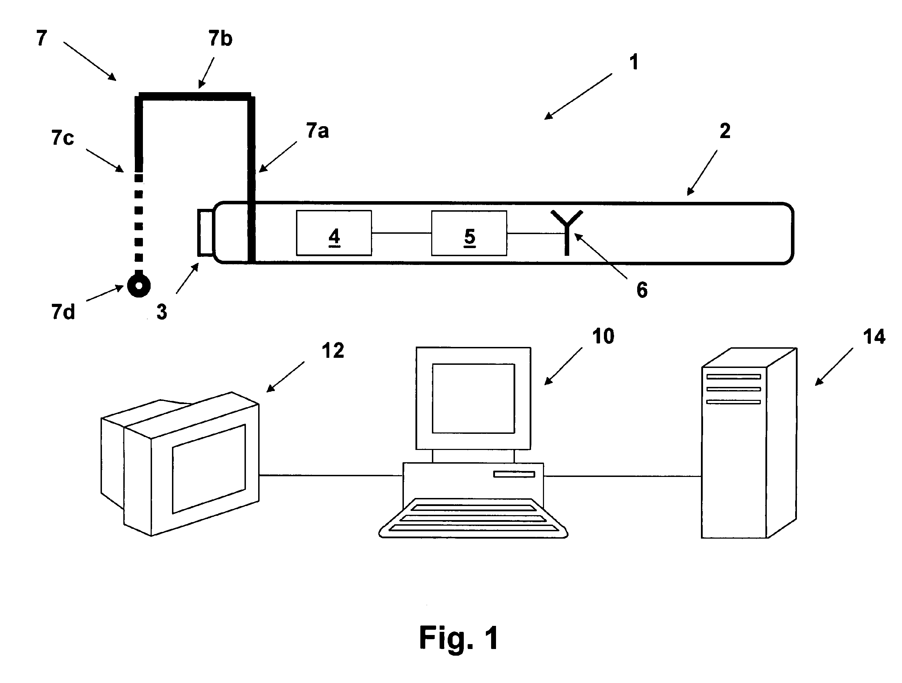

Figure 1 shows an optical dental device 1 according to the present invention. The optical dental device 1 comprises a main body in the form of an elongate handle 2. The handle 2 is shaped so that it can be easily grasped by a dentist using one hand. For instance, the main body can have the shape of a lighting pen. The surface of the handle 2 can be made of a material that allows for a good and comfortable grip.

The handle 2 houses a camera consisting of a lens 3, an image sensor 4, such as a CCD sensor, as well as a control unit 5. Such cameras for an intra-oral use and the necessary components thereof are well known to the person skilled in the art so that these elements will not be described in more detail.

A substantially U-shaped arm member 7 having a first elongate proximal leg 7a, an arch-shaped or transverse transition portion 7b and a second elongate distal leg 7c is with its first proximal leg 7a connected to the distal end of the elongate handle 2 so that the second elongate distal leg 7c of the substantially U-shaped arm member 7 extends at least partially across the field of view of the camera. Although the arm member 7 according to the preferred embodiment is substantially U-shaped the person skilled in the art can readily contemplate various other suitable shapes and configurations thereof that provide for overall dimensions of the optical dental device 1 including the arm member 7 that allow the device 1 to be used as a hand-held device in an intra-oral fashion. Moreover, the person skilled in the art will appreciate that instead of being connected to the distal end of the elongate handle 2 (either fixedly or detachably, such as by means of a clip-connection) the arm member 7 and the elongate handle 2 could be integrally formed.

A plurality of visual markings are disposed along the second distal leg 7c of the substantially U-shaped arm member 7 that starts at the tip 7d thereof and provide for an indication of the distance of a point on the second distal leg 7c of the arm member 7 from the tip 7d thereof. The visual markings, for instance, could comprise a series of patches having a width of about 1 mm and alternating discernible colours, such as black and white. In other words, within a distance of 1 mm from the tip 7d the second distal leg 7c of the arm member 7 could be coloured white, within a distance of 1 mm to 2 mm from the tip 7d the second distal leg 7c could be coloured black, within a distance of 2 mm to 3 mm from the tip 7d the second distal leg 7c could be coloured white again and so forth. The person skilled in the art can readily envisage a multitude of other visual markings having different colours, shapes, widths and so on that provide for the same function as the above-described ones, namely an indication of the distance of a point on the second distal leg 7c of the arm member to the tip 7d thereof, and that fall within the scope of the present invention.

In use, the tip 7d of the second distal leg 7c of the substantially U-shaped arm member 7 can be inserted into a periodontal pocket of a patient until the tip 7d abuts the bottom of the periodontal pocket. This action is captured by the camera and can be displayed on a monitor 12 so that the dentist and possibly also the patient can view the image on the monitor 12. The monitor 12 and the optical dental device 1 are part of a dental examination system that further includes an image processing unit 10 and, optionally, a database 14.

In order for the image processing unit 10 to provide a good determination of the depth of a periodontal pocket, it has to be ensured that the image processing unit 10 performs such a determination by analysing an image taken by the camera at a point in time when the tip 7d of the second distal leg 7c of the substantially U-shaped arm member 7 abuts the bottom of the periodontal pocket being examined.

According to a preferred embodiment, once the tip 7d abuts the bottom of the periodontal pocket with a force above a predefined threshold, such as 0,2 N, the processing of the current image and the determination of the depth of the periodontal pocket is triggered. For measuring the force acting on the tip 7d of the second distal leg 7c or the arm member 7 the optical dental device 1 according to the present invention, preferably, includes a suitably configured force measurement mechanism. For example, the force measurement mechanism can be provided by configuring the tip 7d as a bead connected via a spring element to the second distal leg 7c of the substantially U-shaped arm member 7. That is, the force could be measured by a sensor located in the tip 7d and in communication with the control unit 5. Another suitable sensor would be a sensor based on the piezo-electric effect. However, the person skilled in the art will readily appreciate that other sensors could be employed to determine the force action on the tip 7d or the arm member 7. Alternatively or additionally, the dentist can trigger a measurement, i.e. the analysis of an image by the image processing unit 10, using a button (not shown) provided on the elongate handle 2 of the optical dental device 1 according to the present invention or an audible command to be received by the image processing unit 10.

The image processing unit 10 is configured to automatically analyze the images provided by the camera, for instance by means of a suitably configured software. By means of the visual markings disposed on the second distal leg 7c of the substantially U-shaped arm member 7 the image processing software is capable of determining the depth of a periodontal pocket on the basis of an image that shows how far the second distal leg 7c of the substantially U-shaped arm member 7 is inserted into such a periodontal pocket.

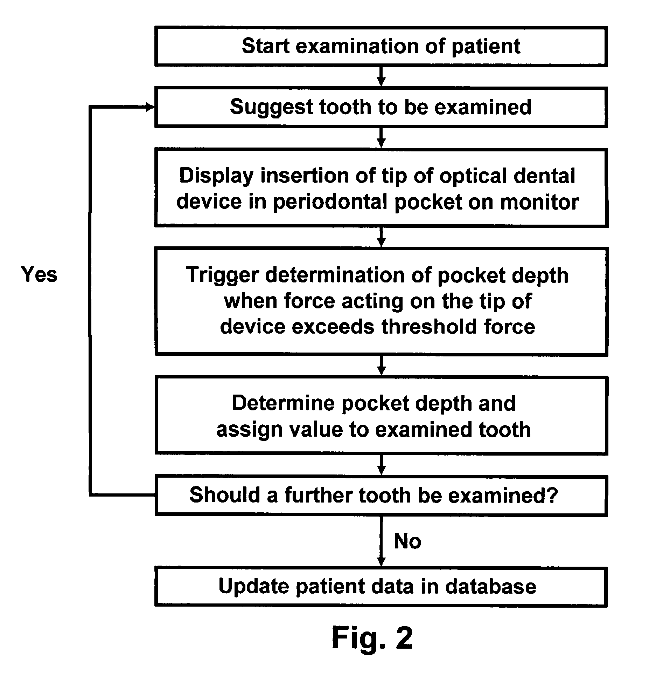

Under additional reference to figure 2 a preferred method of operating the optical dental device 1 according to the present invention in the context of the dental examination system will be described. Upon starting a new examination of a patient, the dental examination system will suggest the dentist a tooth to be examined. The corresponding information could be transmitted to the distance via a visual display on the monitor 12 and/or via an audible message produced by the image processing unit 10. Alternatively, the dentist could also inform the dental examination system about which tooth is going to be examined first. It is also contemplated that the dentist just starts the examination and informs the dental examination system about the examined tooth only during or after the examination.

The insertion of the tip 7d of the second distal leg 7c of the substantially U-shaped arm member 7 into a periodontal pocket of the tooth being examined is continuously imaged by the camera and the captured images are transmitted via an antenna 6 to the image processing unit 10 and/or the monitor 12. This allows the dentist and possibly also the patient to follow the insertion process of the tip 7d of the second distal leg 7c of the substantially U-shaped arm member 7 into a periodontal pocket of the tooth being examined on the monitor 12.

As already described further above, once the tip 7d abuts the bottom of the periodontal pocket of the tooth being examined with a force above a predefined threshold, the processing of the current image and the determination of the depth of the periodontal pocket is triggered. That is, the image processing unit will analyse the current image and determine on the basis of the visual markings on the second distal leg 7c of the substantially U-shaped arm member 7 the distance of a point on the second distal leg 7c from the distal tip 7d thereof, wherein the point on the second distal leg 7c is characterized by being the point on the second distal leg 7c closest to the distal tip 7d that is not covered by gum tissue surrounding the periodontal pocket of the tooth being examined.

Once the image processing unit 10 has determined a value for, i.e. "measured", the depth of the periodontal pocket of the tooth being examined, the dentist can either stop the examination or continue with a second tooth to be examined. In case the examination should continue, the image processing unit suggests another tooth to be examined and the above-described steps are repeated with respect to this second tooth and so on. After all teeth to be examined have been examined, the "measured" depths of the periodontal pockets of the teeth that have been examined can be stored in the database 14 connected to the image processing unit 10 in order to update the patient data.

The present invention as described in detail above is not limited to the particular devices, uses and methodology described as these may vary. For instance, the person skilled in the art will appreciate that although the monitor and the image processing unit of the dental examination system according to the present invention have been described as separate units, these units could also be integrated into a single unit.

It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

## Claims

1. An optical dental device (1), comprising:
an elongate handle (2) for manually handling the optical dental device (1);
a camera integrated in the distal end of the elongate handle (2);
an arm member (7) connected to the elongate handle (2) so that a distal elongate portion (7c) of the arm member (7) extends at least partially across the field of view of the camera (2),
wherein a plurality of visual markings are disposed on and along the distal elongate portion (7c) of the arm member (7) providing information about the distance of a point of the distal elongate portion (7c) of the arm member (7) from the tip (7d) thereof.

2. The optical dental device (1) of claim 1, wherein the arm member (7) is substantially U-shaped and comprises a first proximal leg (7a) and a second distal leg (7c).

3. The optical dental device (1) of claim 1, wherein the tip (7d) of the distal elongate portion (7c) of the arm member (7) is configured as a bead with a diameter of about 0,5 mm.

4. The optical dental device (1) of claim 1, wherein the camera comprises a lens (3) and an image sensor (4).

5. The optical dental device (1) of claim 1, further comprising a control unit (5) for controlling the camera and transmitting images captured by the camera via an antenna (6).

6. The optical dental device (1) of claim 1, further comprising selection means for allowing a user to select the current image captured by the camera.

7. The optical dental device (1) of claim 6, wherein the selection means are a button located on the elongate handle (2).

8. The optical dental device (1) of claim 1, further comprising a force measurement sensor for measuring the force acting on the tip (7d) in an abutting relationship with the bottom of a periodontal pocket.

9. The optical dental device (1) of claim 8, wherein the force measurement sensor is a piezoelectric sensor located in the tip (7d).

10. A dental examination system, comprising:
the optical dental device (1) according to any one of the preceding claims;
an image processing unit (10) for receiving and processing images captured by the camera of the optical dental device (1); and
a monitor (12) for displaying images captured by the camera of the optical dental device (1).

11. The dental examination system of claim 10, wherein the dental examination system further comprises a database (14) connected to the image processing unit (10) for storing image and patient data.

12. Method of operating a dental examination system, comprising the following steps:
a) displaying the insertion of the tip (7d) of an optical dental device (1) in a periodontal pocket of a tooth being examined on a monitor (12), wherein the optical dental device (1) comprises:
an elongate handle (2) for manually handling the optical dental device (1);
a camera integrated in the distal end of the elongate handle (2); and
an arm member (7) connected to the elongate handle (2) so that a distal elongate portion (7c) of the arm member (7) extends at least partially across the field of view of the camera (2),
wherein a plurality of visual markings are disposed on and along the distal elongate portion (7c) of the arm member (7) providing information about the distance of a point on the distal elongate portion (7c) of the arm member (7) from the tip (7d) thereof;
b) triggering the determination of the depth of the periodontal pocket of the tooth being examined; and
c) determining the depth of the periodontal pocket of the tooth being examined by determining the distance between the tip (7d) and the point of the distal elongate portion (7c) of the arm member (7) closest to the tip (7d) that is not covered by the adjacent gum.

13. Method of operating a dental examination system according to claim 12, wherein the determination of the depth of the periodontal pocket of the tooth being examined is triggered by the dentist using a button or an audible command.

14. Method of operating a dental examination system according to claim 12, wherein the determination of the depth of the periodontal pocket of the tooth being examined is triggered when the force acting on the tip (7d) of the distal portion (7c) of the arm member (7) is measured to exceed a predefined threshold force.

15. Method of operating a dental examination system according to claim 12, wherein the method comprises the further step of suggesting a tooth to be examined prior to step a).
